# FASCICULE DE BREVET EUROPEEN

(11) **EP 0 683 680 B1**
(45) Date de publication et mention de la délivrance du brevet: **29.08.2001**
(21) Numéro de dépôt: 95903390.3
(22) Date de dépôt: 09.12.1994
(51) Int. Cl.: A61L 27/00

(54) **MATERIAU DE SUBSTITUTION DES PARTIES MOLLES TISSULAIRES OU MUSCULAIRES, SON PROCEDE DE FABRICATION, ET SON APPLICATION A LA REALISATION DE GREFFES**
MATERIAL ZUM ERSATZ VON WEICHTEILGEWEBEN ODER MUSKELN, VERFAHREN ZU SEINER HERSTELLUNG UND SEINE ANWENDUNG IN PROTHESEN
REPLACEMENT MATERIAL FOR SOFT PARTS OF TISSUES OR MUSCLES, METHOD FOR MAKING SAME, AND USE OF SAID MATERIAL IN GRAFTS

(30) Priorité: 10.12.1993 FR 9314962
(43) Date de publication de la demande: 29.11.1995
(73) Titulaire: FORTUNE BASE MANAGEMENT, LTD., Central Hong Kong (HK)
(72) Inventeur: Camprasse, Georges, 77480 Villenauxe-la-Petite (FR); Camprasse, Serge, 77500 Chelles (FR)
(74) Mandataire: Thinat, Michel
(86) Numéro de dépôt international: FR9401446
(87) Numéro de publication internationale: WO9515773

(56) Documents cités:
- DE-A- 3 038 319
- PATENT ABSTRACTS OF JAPAN vol. 013, no. 461 (C-645) 18 Octobre 1989 & JP,A,01 180 809 (NOEVIR CO LTD) 18 Juillet 1989

## Description

Le produit selon l'invention concerne un biomatériau obtenu après traitement spécifique de Spongia Officinalis ou tout autre Porifère, destiné à remplacer les pertes de substance tissulaire ou musculaire consécutives à un traumatisme, à une exérèse chirurgicale, et à une destruction tumorale ou dégénérative.

En effet, on sait que Spongia Officinalis fait partie de la classe des démosponges, dont le squelette est formé de fibres de spongine, le corps d'un ensemble de cellules qui ne sont pas groupées en tissu. La caractéristique essentielle des éponges est l'absence de différentiation cellulaire, ce qui explique leur faculté de régénération.

Le corps des éponges est formé de cellules appartenant à un petit nombre de types différents concourrant à la formation de véritables unités fonctionnelles, il est dépourvu d'organe.

Les pertes de substance provoquent généralement non seulement des dommages esthétiques mais aussi fonctionnels. La réparation de tels dommages se fait chirurgicalement soit par des greffes dermo-cutanées, musculaires ou prothétiques.

Les greffes dermo-cutanées nécessitent la prise d'un greffon préparé sous forme de boudin pédiculé à partir d'un territoire non exposé et dont la mise en oeuvre demande plusieurs semaines ; de plus, la prise de ce greffon est un acte chirurgical contraignant.

Les greffes musculaires se font par transposition de faisceaux musculaires prélévés par translation sur des muscles avoisinant la perte de substance.

Les réparations par substitution prothétique sont généralement le fait de prothèses en silicone.

Toutefois, tous ces procédés de réparations tissulaires présentent de nombreux inconvénients. Pour les greffes dermo-cutanées, des séquelles cicatricielles inesthétiques au niveau du greffon. Les greffes par translation des faisceaux musculaires provoquent des séquelles esthétiques et fonctionnelles nécessitant une longue et pénible rééducation. Quant aux réparations par ajonction prothétique elles peuvent être le siège de réactions inflammatoires donnant lieu à la formation de coques fibreuses ainsi que des réactions allergiques pouvant conduire à la dépose de la prothèse.

Il fallait donc trouver un biomatériau qui permette une reconstruction tissulaire rapide par réhabitation et ce qui fait l'objet de l'invention.

Après battage et lavage sous courant d'eau à une pression de 2 bars, destinés à le débarasser des microorganismes qu'il pourrait contenir, le produit selon l'invention est obtenu après traitement spécifique thermo-chimique sous haute atmosphère destiné à neutraliser le code génétique, à modifier la perméabilité membranaire des cellules du Porifère et à l'inactivation bactérienne et virale.

Ce traitement thermochimique a pour but d'obtenir :
(1) la décontamination bactérienne et virale du Porifère,
(2) l'élimination des pigments contenus dans le Porifère,
(3) la modification de la perméabilité membranaire, et
(4) la neutralisation de code génétique du Porifère.

Un exemple d'un traitement thermochimique préférentiel réalisé sur Spongia Officinalis consiste en l'immersion de Spongia Officinalis dans un bain d'hypochlorite de sodium (NaClO) ou de toute autre solution acido-basique, portée à ébullition sous pression de 2 bars à 200°C pendant 2 fois 2 heures après changement du bain. Après lavage sous pression par flux continu d'eau démineralisée à 60°C, le produit selon l'invention est traité par immersion dans un bain de permanganate de potassium (KMnO₄) pendant 1 heure à 80°C puis lavé sous flux continu d'eau démineralisée à 60°C et traité par H₂O₂/H₂O distillée en cycle alterné pendant 2 heures. Après lavage de 1 heure sous courant d'eau le produit selon l'invention est séché sous courant d'air chaud par passage en étuve pendant 2 heures par paliers successifs de 15°C en 15°C jusqu'à 70°C. Le produit selon l'invention est conditionné sous vide dans un moule anatomique de manière à obtenir des formes préétablies puis stérilisé par traitement ionisant (rayonnements β ou γ à 2,5 mégarads).

Selon un mode de réalisation préférentiel le produit selon l'invention est préformé de manière à remplacer une perte de substance de la partie antérieure de la cuisse, du mollet, du bras, de la région fessiaire, pectorale, faciale, et plus généralement toute perte de substance en chirurgie réparatrice et esthétique.

Les exemples suivants illustrent les applications du produit selon l'invention en chirurgie réparatrice, sans que ceux-ci soient limitatifs.

Après section et exérèse, sous anesthésie générale, d'une portion de 5 cm du muscle prévertébral chez le mouton, le produit selon l'invention est placé dans la perte de substance et recouvert par l'aponévrose musculaire qui est suturée. Dans un délai de 12 semaines la pièce anatomique est prélevée et montre à l'examen macroscopique un tissu musculaire régénéré et parfaitement vascularisé. L'examen microscopique met en évidence la néoformation de fibres conjonctives ayant pénétré les foramen du produit selon l'invention s'enchevêtrant en formant des faisceaux en continuité avec ceux des fibres musculaires du site receveur, se servant des fibres de spongine comme substrat.

Dans un second mode de réalisation, le produit selon l'invention associé à un gel de collagène non réticulé est placé en position infra-glandulaire dans la région mammaire d'une brebis de façon à augmenter le volume de la glande. Dans un délai de 12 semaines on constate la persistance de l'augmentation de volume, et à la palpation, l'absence de coque fibreuse. Le produit selon l'invention peut être associé à l'amidon, l'alginate, ou l'albumine, ou toutes autres substances biocompatibles, qui auraient la propriété de modifier la consistance et la tenue de la prothèse faite du produit selon l'invention.

L'examen anatomopathologique confirme l'existence d'un tissu conjonctif dense richement vascularisé, non inflammatoire ainsi que l'absence de fibrocyte.

Toutes ces expérimentations mettent en évidence la non toxicité du produit selon l'invention, sa parfaite biocompatibilité et son rôle de matrice pour les cellules et les fibres conjonctives qui se servent des fibres de spongine.

Il appartiendra à l'homme du métier de mettre en oeuvre le produit selon l'invention issu de Spongia Officinalis ou tout autre Porifère, chaque fois qu'il aura besoin qu'un biomatériau pour la reconstruction tissulaire ou musculaire.

## Revendications

1. Utilisation de Porifère rendu biocompatible pour la fabrication d'un matériau de substitution des parties molles tissulaires ou musculaires.

2. Utilisation selon la revendication 1, **caractérisée en ce que** le Porifère est Spongia Officinalis.

3. Utilisation selon la revendication 1 ou 2, **caractérisée en ce que** le Porifère est associé à un autre produit biocompatible.

4. Utilisation selon la revendication 3, **caractérisée en ce que** le produit biocompatible est choisi parmi l'amidon, l'albumine, un alginate et du collagène non réticulé.

5. Utilisation selon l'une des revendications précédentes, **caractérisée en ce que** le Porifère est rendu biocompatible par:
- battage et lavage sous courant d'eau,
- traitement thermo-chimique sous haute atmosphère,
- séchage sous courant d'air chaud, et
- traitement par rayonnement ionisant.

6. Utilisation selon l'une quelconque des revendications précédentes, **caractérisée en ce que** le Porifère est moulé sous vide conformément à la forme anatomique de la matière à remplacer.

7. Utilisation selon l'une quelconque des revendications précédentes, pour la réalisation de greffes dermo-cutanées ou musculaires.

## Patentansprüche

1. Einsatz von biokompatibel gemachten Schwammtieren zur Herstellung eines Materials zum Ersatz von Weichteilgewebe oder Muskeln.

2. Einsatz gemäß Anspruch 1, **dadurch gekennzeichnet**, daß das Schwammtier Spongia Officinalis ist.

3. Einsatz gemäß Anspruch 1 oder 2, **dadurch gekennzeichnet**, daß das Schwammtier einem anderen biokompatiblen Produkt zugeordnet wird.

4. Einsatz gemäß Anspruch 3, **dadurch gekennzeichnet**, daß das biokompatible Produkt aus Stärke, Albumin, einem Alginat und einem nicht vernetzten Kollagen ausgewählt wird.

5. Einsatz gemäß einem der vorgenannten Ansprüche, **dadurch gekennzeichnet**, daß das Schwammtier biokompatibel gemacht wird durch:
- Ausklopfen und Waschen unter fließendem Wasser,
- thermo-chemische Behandlung unter hoher Atmosphäre,
- Trocknen unter Heißluft und
- Behandlung durch lonenstrahlung.

6. Einsatz gemäß einem der vorgenannten Ansprüche, **dadurch gekennzeichnet**, daß das Schwammtier gemäß der anatomischen Form des zu ersetzenden Materials unter Vakuum geformt wird.

7. Einsatz gemäß einem der vorgenannten Ansprüche zur Realisierung einer Hautplastik der Lederhaut oder einer Muskelplastik.

## Claims

1. Use of Poriferous which is made biocompatible for manufacturing a replacement material for soft parts of tissues or muscles.

2. Use according to claim 1, **characterized in that** the Poriferous is Spongia Officinalis.

3. Use according to claim 1 or 2, **characterized in that** the Poriferous is associated with another biocompatible product.

4. Use according to claim 3, **characterized in that** the biocompatible product is chosen among starch, albumin, an alginate and a non-crosslinked collagen.

5. Use according to one of the preceding claims, **characterized in that** the Poriferous is made biocompatible by :
- beating and washing under a water stream,
- thermo-chemical treatment under high atmosphere,
- drying under an hot air stream, and
- treating by ionizing radiation.

6. Use according to anyone of the preceding claims, **characterized in that** the Poriferous is vacuum moulded according to the anatomical shape of the material to be replaced.

7. Use according to anyone of the preceding claims for making dermo-cutaneous or muscular grafts.
